# EUROPEAN PATENT SPECIFICATION

(11) **EP 2 944 944 B1**
(45) Date of publication and mention of the grant of the patent: **20.10.2021**
(21) Application number: 15166609.6
(22) Date of filing: 06.05.2015
(51) Int. Cl.: G01N 21/3504, G01N 21/31, G01N 33/00, G01N 21/39

(54) **GAS DETECTOR AND METHOD OF DETECTION**
GASDETEKTOR UND VERFAHREN ZUR DETEKTION
DÉTECTEUR DE GAZ ET PROCÉDÉ DE DÉTECTION

(30) Priority: 12.05.2014 IN CH23772014
(43) Date of publication of application: 18.11.2015
(73) Proprietor: General Electric Company, Schenectady, NY 12345 (US)
(72) Inventor: Mitra, Chayan, 560066 Bangalore (IN); Joshi, Narendra Digamber, Niskayuna, NY 12309 (US); Tilak, Vinayak, 560066 Bangalore (IN); Smith, Gordon, Schenectady, NY 12345 (US); Fung, Eric YuHang, Houston, TX 77015 (US); Maity, Sandip, 560066 Bangalore (IN); Sharma, Rachit, 560066 Bangalore (IN); Handelsman, Steven Keith, West Chester, OH 45069 (US)
(74) Representative: BRP Renaud & Partner mbB Rechtsanwälte Patentanwälte Steuerberater

(56) References cited:
- WO-A1-2014/109126
- DE-B3-102009 025 147
- JP-A- 2007 309 800
- JP-A- 2012 026 918
- JP-A- 2013 117 517
- US-A1- 2007 045 542
- US-A1- 2008 144 677
- US-A1- 2012 033 220
- US-A1- 2012 307 241
- US-A1- 2013 056 626
- US-A1- 2013 292 571

## Description

### BACKGROUND OF THE INVENTION

The invention relates generally to a gas detector and more particularly to a gas detector for power plant emission measurements and control.

Gas detectors have been used in various industrial applications such as power plants, transportation vehicles, gas turbines, and industrial, environmental, and biological processes and installations. A gas detector typically detects the presence of one or more gases in a given area. In some applications, detectors can further measure concentrations of specific gases.

In recent years, utility deregulation and increasing electrical demand have led to the integration of solar and wind power with combined cycle (CC) power plants. This integration has led to a desire to reduce start-up and shutdown times for the gas turbines in the CC power plants. The emission levels from a gas turbine vary depending on the manner in which the machine starts up or shuts down. Often, emissions from power plants may deteriorate as a result of seasonal changes, changes in fuel quality, and deterioration of gas turbine airfoils. A continuous emission measurement system (CEMS) comprising a gas detector measures gaseous emission species such as NOX (for example Nitrogen monoxide NO and Nitrogen dioxide NO2), carbon monoxide (CO) and ammonia (NH3) for continually demonstrating compliance to permitted emission limits.

A CEMS coupled with a gas detector typically uses an extractive process of sampling out near homogeneous portions of the exhaust gas from the exhaust gas duct, a heat recovery steam generator (HRSG) coupled to the exhaust gas duct, and a gas exhaust stack. This process typically requires from about 30 seconds to about 3 minutes of measurements as well as frequent purging and calibration processing. A CEMS typically uses measurement techniques such as interaction of light with the emission species like light absorption, chemi-luminescence, and/or non-dispersive infrared analysis. In light absorption based techniques, light beams of particular wavelengths, at which the respective emission species to be detected have peak absorption, are transmitted through an area where the emission species are expected to be present.

One or more photo detectors then receive the transmitted light beams. Various types of photo detectors are available commercially and are chosen based on the wavelength of the light beams employed in detecting the gases. By employing Bear-Lambert's law and other correction and conversion factors, a controller can convert the photo detector output signal to provide concentration information of the specific gas or gases of interest and may further control and manage emissions.

Power generation plants require reliable, accurate, and timely detection and measurement of multiple gases. However, the harsh and challenging operating conditions of power generation plants may adversely affect selectivity and sensitivity in light absorption spectroscopy measurements. For example, during transient operations rapid changes of NOx levels can occur, and the CEMS technology will introduce a time delay in the NOx measurement. In such situations, ammonia injection may be incorrectly high or low depending on the actual NOx change during the time delay. A high ammonia injection in selective catalytic reduction (SCR) system leads to the formation of corrosive ammonium sulfates adversely impacting downstream hardware. A low ammonia injection in SCR system may lead to unnecessarily high stack NOx because not enough ammonia was injected when needed.

In addition, many challenges exist for in-situ monitoring of power plant exhaust emission species such as the presence of moisture, harsh environment, high temperatures, and dynamic fluctuations in trace gas concentrations.

JP2007309800, JP2013117517, and JP2012026918 disclose the systems for measuring gas concentrations using optical means. In particular, JP2013117517 discloses a gas detector with a mid-infrared light source and a near-infrared light source, whose beams are coupled by a coupling unit and directed through a gas flow path between a first and second window towards a receiver unit.

US 2012/0033220 A1 discloses a system for detecting chemicals at long-range distances using a laser system and retroreflectors. DE 10 2009 025 147 B3 discloses a gas detector with light sources and a retroreflector. US 2007/0045542 A1 discloses a system with a light source and a retroflector.

Accordingly, there is a need for simultaneous, in-situ, real time, selective, and sensitive measurement of multiple emission species to reduce and control startup and transient emissions in power plants. Further it would be desirable to provide reliable measurements with greater accuracy, repeatability, and consistency to demonstrate compliance or attainment to regulatory emission levels.

### BRIEF DESCRIPTION OF THE INVENTION

The invention is defined by the independent claims.

### DRAWINGS

These and other features, aspects, and advantages of the present invention will become better understood when the following detailed description is read with reference to the accompanying drawings, in which like characters represent like parts throughout the drawings, wherein:
FIG. 1 is block diagram of a gas detection system in accordance with a non-claimed example.
FIG. 2 is block diagram of a gas detection system in accordance with another non-claimed example.
FIG. 3 is a block diagram of a receiver unit in accordance with another non-claimed example.
FIG. 4 is a block diagram in accordance with an exemplary embodiment of the present invention.
FIG. 5 is a. schematic representation of a gas turbine engine, a heat recovery steam generator (HRSG) with a selective catalytic reduction (SCR) system, and a control configuration according to an exemplary embodiment of the invention.
FIG. 6 is a flowchart of a gas detection method comprising the present invention.
FIG 7 is a flowchart of an example calibration method for carbon monoxide (CO) monitoring light source.

### DETAILED DESCRIPTION

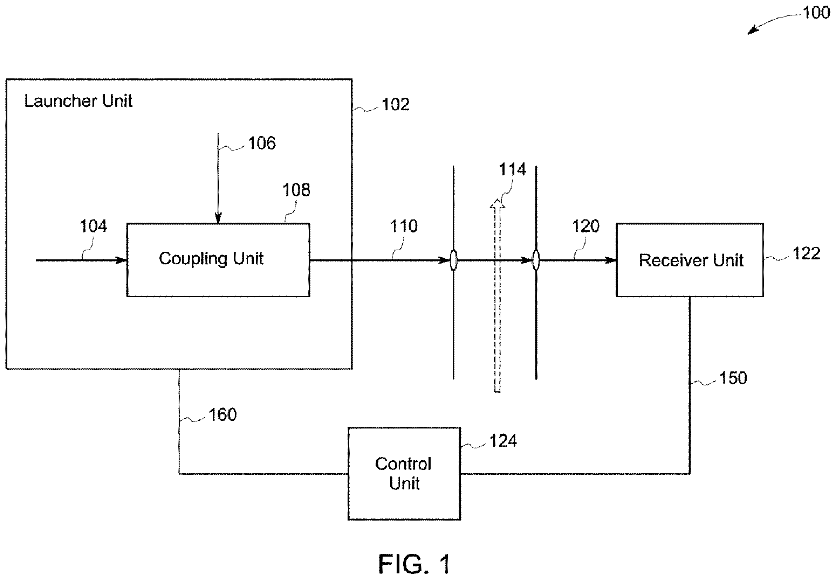

FIG. 1 illustrates a gas detector 100 comprising a launcher unit 102 for coupling a light beam 104 in the mid-infrared (mid-IR) wavelength range with a light beam 106 in the infrared (IR) wavelength range in a coupling unit 108 for providing a coupled and merged single light beam 110 and directing the single light beam 110 towards a gas flow path 114. A receiver unit 122 collects the light beam 120 transmitted through the gas flow path, detects at least two gases present in the gas flow path 114, and generates one or more photo detector current signals 150. In more conventional embodiments wherein multiple light beams are transmitted through and received from a gas flow path, challenges associated with multiple light beams traveling through different regions in the gas flow path result in potential inaccuracies in measurements due to the turbulent transmission path and beam steering. Use of a single light beam, as described in this embodiment, removes such challenges, A control unit 124 processes the one or more photo detector current signals to measure concentrations of the at least two gases present in the gas flow path. Control unit 124 may further generate one or more driver current signals 160. In one embodiment, driver current signals 160 may be used to correct any misalignment of the launcher unit. Although a single control unit is shown as a separate block in FIG. 1 for purposes of illustration, in some embodiments, the control unit may comprise one or more units and may be situated in the receiver unit and/or the launcher unit.

In the more specific example of FIG, 2, the launcher unit 102 further includes a first set of light sources 202 to emit light beams in the mid-infrared wavelength range, a first light transmission network 204 for bundling the light beams from the first set of light sources to produce a bundled first light beam 104 in the mid-IR wavelength range, a second set of light sources 212 to emit light beams in an infrared wavelength range, and a second light transmission network 214 for bundling the light beams from the first set of light sources to produce a bundled second light beam 106 in the IR wavelength range. The coupling unit 108 in the launcher unit merges the mid-IR and IR light beams 104 and 106 transmitted respectively through the first and second light transmission networks 204 and 214 into the single light beam 110. The single light beam 110 then is transmitted through a launching window 112 towards a gas flow path 114. The single light beam 110 interacts with gaseous species in the gas flow path as it travels through a path 116 inside the gas flow path and towards a receiving window 118. In one example the interaction of the single light beam with gaseous species in the gas flow path includes light absorption. After interacting with the gaseous species, the single light beam 120 emerges from the receiver window and is transmitted towards the receiver unit 122. The receiver unit comprises photo detectors sensitive to specific wavelength ranges selected based on the requirement for detecting specific emission species. A control unit 124 receives photo detector current signals 150 from the receiver unit, uses the photo detector current signals to measure the emission species, and, in one embodiment, provides the driver current signal 160 to the first and second sets of light sources.

As used herein, "mid-infrared wavelength range" includes wavelengths ranging from about 3.5 µm to about 20 µm. Mid-IR light wavelength ranges in the electromagnetic radiation spectrum comprise absorption features of many molecules including water molecules and gaseous species in a gas turbine power plant such as nitrogen dioxide, nitrogen monoxide, carbon monoxide, and ammonia. In the invention, the first set of light sources for emitting mid-infrared light beams are configured such that one of the light beams has a different wavelength than at least one other of the light beams. For example, it is often desirable to simultaneously detect nitrogen dioxide and nitrogen monoxide, the most common gaseous emission species in a power plant, at two separate wavelengths to ensure selectivity and sensitivity. With respect to particular wavelengths of interest, nitrogen dioxide (NO2) may be detected near at least one of about 3.5 µm and about 6.3 µm, nitrogen monoxide (NO) may be detected near at about 5.3 µm, ammonia may be detected in the mid-IR wavelength range near at least one about 9 µm and about 10.4 µm, and carbon monoxide may be detected in the mid-IR wavelength range near 4.86 µm. In one embodiment two or more of such gases may be simultaneously detected such that one of the light sources transmits at a wavelength targeted towards a first gas and at least one other of the light sources transmits at a wavelength targeted towards at least one other gas.

In an exemplary embodiment, the first set of light sources 202 comprises a plurality of quantum cascade lasers (QCLs). Some of the advantages of such lasers include high optical power output of the order of several hundred milliwatts, broad tuning range, ability to operate at room temperature, large dynamic range, and failsafe operation combined with solid-state reliability. Quantum cascade lasers such as distributed feedback quantum cascade lasers (DFB-QCL) may be operated in a single mode allowing rapid scanning of a spectral wavelength range, resulting in gas detection measurement within about 1 second or within about 5 seconds. In a more specific embodiment, the first set of light sources 202 may include three QCLs emitting light beams at about 5.3 µm, about 6.3 µm and about 9 µm corresponding respectively to the peak absorption of nitrogen monoxide (NO), nitrogen dioxide (NO2) and ammonia (NH3). In an alternative embodiment, the first set of light sources 202 may include a single wavelength light source and a wavelength converter for converting the single wavelength light beam into a light beam comprising multiple mid-IR wavelengths.

As used herein, "infrared wavelength range" includes wavelengths ranging from about 0.8 µm to about 3 µm. Infrared (IR) light wavelength ranges of the electromagnetic radiation spectrum comprise absorption features of many molecules including water molecules and gaseous species in a power plant such as ammonia, carbon monoxide, and carbon dioxide. In the invention, second set of light sources 212 are configured for emitting infrared light beams with one of the light beams having a different wavelength than at least one other of the light beams. For example, the second set of light sources 212 may be configured for emitting one of the light beams near or at a peak absorption wavelength of carbon monoxide, and one other light beam near or at a wavelength insensitive to one or more emission gaseous species in the gas flow path. It is useful if the other wavelength is in additionally insensitive to water molecules. In one more specific aspect of this example, the second set of light sources 212 may be configured for emitting the one other light beam near or at a wavelength insensitive to carbon monoxide (CO) or water (H2O) molecules. In such embodiments, misalignments of the launcher and receiver units may be monitored as described in commonly assigned US20120307241. In an exemplary embodiment, the second set of light sources 212 may include a near-IR laser in a standard butterfly package, emitting light beam at about 2.3 µm corresponding to the peak absorption wavelength of carbon monoxide (CO) and one other light source in the second set emitting at about 1.3 µm, a wavelength at which the light absorption by both CO and H2O molecules is low. The second set of light sources 212 may include, for example, one or more of light emitting diodes, laser diodes, fabry-perot lasers, or any other portable light sources.

In some embodiments, the first light transmission network 204 may include light to fiber couplers (not shown) for coupling light beams from each of the first set of light sources 202 to an individual hollow-core optical fiber in the fiber optic network 206. In one embodiment, the first light transmission network may further include hollow core fibers bundled together for forming a bundled fiber 208. In some embodiments, the bundled fibers are coupled to a beam collimator 210 for enhancing beam convergence and for transmitting the bundled beams 104 towards a coupling unit 108. In an exemplary embodiment, the total length of the hollow-core fibers may be in the range of about 20 centimeters to about 30 centimeters..

In some embodiments, the second light transmission network 214 may include individual optical fibers 216 coupled to respective light source in the second set of light sources 212. In one embodiment, the optical fibers 216 are attached to a beam collimator 218 for enhancing beam convergence and for transmitting the light beams 106 from second set of light sources 212 towards a coupling unit 108 and beam collimators. In some embodiments, the second light transmission network may comprise a network similar to that of the first transmission network.

In one specific embodiment, the coupling unit 108 comprises a dichroic mirror. In an exemplary embodiment, the coupling unit comprises a semitransparent dichroic mirror to couple the mid-IR and IR light beams. As further examples, dichroic mirror 218 in the coupling unit may comprise a semitransparent planar mirror or a semitransparent parabolic mirror or any other semitransparent curved mirror. In the parabolic example, the semitransparent parabolic dichroic mirror 218 may receive the mid-IR light beams 104 and IR light beams 106 on its convex and concave surfaces respectively. As a result, the mid-IR light beams transmit through the semitransparent dichroic mirror and the IR light beams reflect, resulting in a single coupled and merged light beam 110 comprising both mid-IR and IR wavelengths.

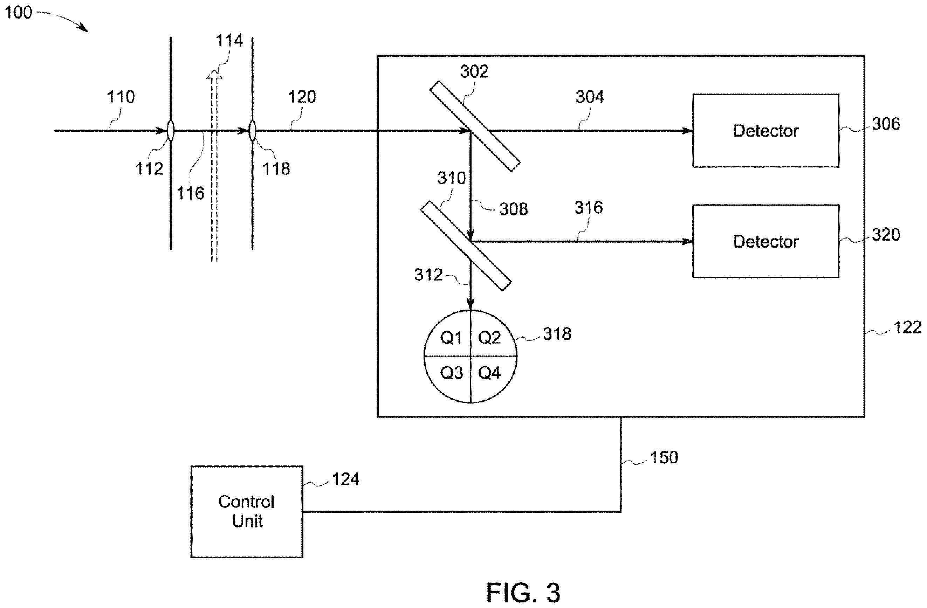

FIG. 3 illustrates an exemplary receiver unit 122 that receives the single coupled and merged beam 120 transmitted through the gas flow path. In one embodiment the receiver unit comprises a dichroic beam splitter 302 that receives the single coupled and merged beam 120. The dichroic beam splitter transmits a portion of the light beam 120 comprising mid-IR wavelengths 304 towards a mid-IR photo detector 306 and reflects another portion of the light beam comprising IR wavelengths 308 towards a second dichroic beam splitter 310. The second dichroic beam splitter 310 transmits a first light beam 312 towards a first IR photo detector 318 and reflects another light beam 316 having higher wavelength than the first light beam towards another IR photo detector 320.

In an exemplary embodiment, the dichroic beam splitter 302 comprises a Zinc Selenide (ZnSe) beam splitter, and the mid-IR photo detector 306 comprises a mercury-cadmium-telluride (MCT or Hg-Cd-Te) detector. In some embodiments, a chiller unit (not shown) may be attached to the mid-IR MCT detector for improving the detectivity and lowering the dark current of the detector. In an exemplary embodiment the MCT detector operates in a photovoltaic mode.

In an exemplary embodiment, the dichroic beam splitter 310 comprises a calcium fluoride (CaF) beam splitter, the IR photo detector 320 comprises a InGaAs photo detector, and the IR photo detector 318 comprises a silicon quad photo detector. In an exemplary embodiment, the light beam 312 comprises a monitor beam used for detecting and correcting launcher and receiver misalignments. In some other embodiments, which may be alternatives or in addition to the misalignment detection embodiment, the light beam 312 may detect issues with window fogging and/or be used to improve the signal to noise ratio of the gas detector.

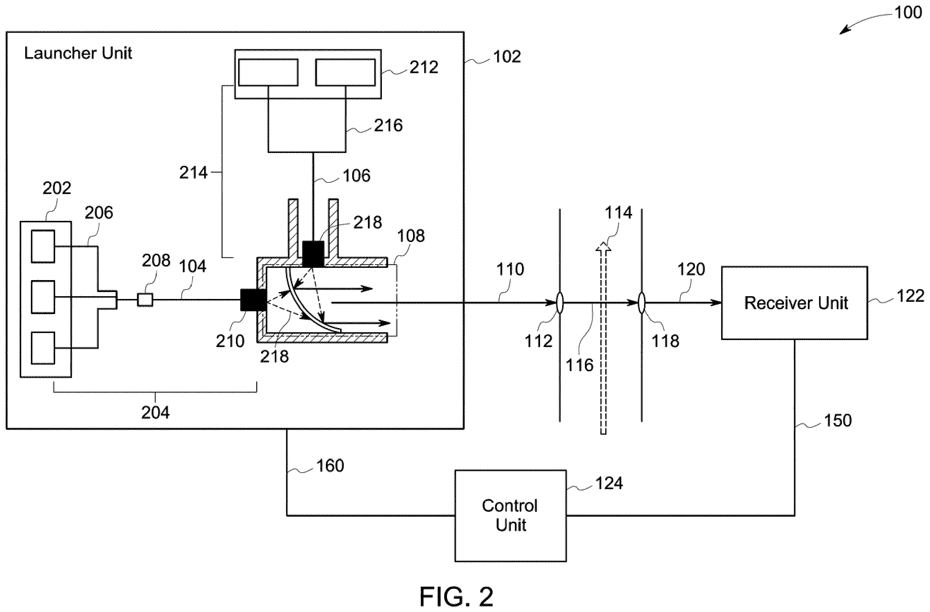

In some embodiments, the control unit 124 includes a signal processing technique that processes the photo detector current signals for determining concentration of at least two gaseous emission species selected from a list comprising NO2, NO, CO and NH3. The concentrations may be determined using signal processing techniques such as for example, wavelength modulation spectroscopy, frequency modulation spectroscopy, and direct absorption spectroscopy as described in commonly assigned US20100091278A1. In some embodiments, the control unit 124 processes the photo detector current signals from the photo detectors 306, 310 and 320 and uses them to determine current drive signals for the launcher unit 102 (FIGs. 1-2).

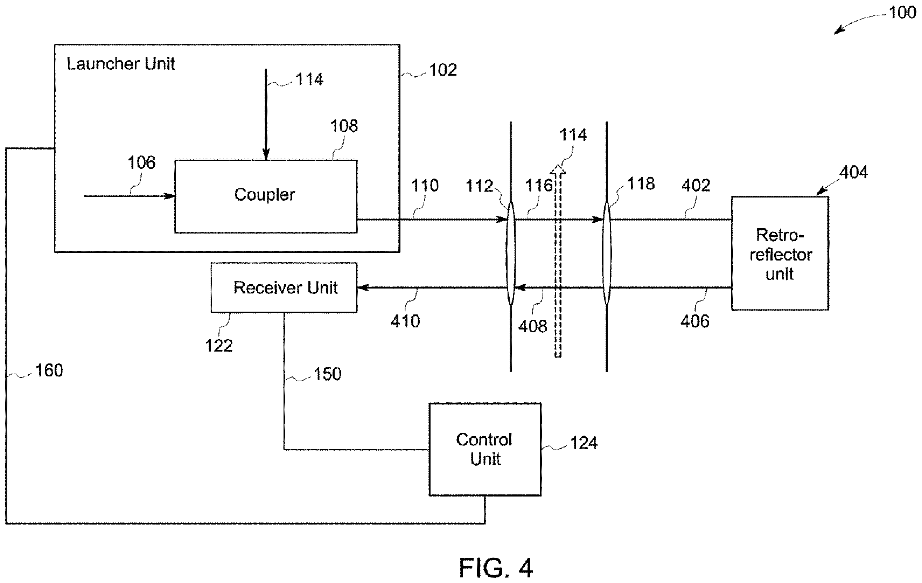

Fig. 4 illustrates the invention, wherein the launcher unit 102 and the receiver unit 122 are situated on a first side of the gas flow path, and a retro-reflector unit 404 is situated on a second side of the gas flow path opposite the first side. In the invention, the single light beam 110 interacts with gaseous species in the gas flow path as it travels through a path 116 inside the gas flow path and towards the window 118. After interacting with the gaseous species, the single light beam 402 emerges from the window 118 and is transmitted towards the retro-reflector 404 and undergoes two or more reflections in the retro-reflector 406. The light beam 406 emerges from the retroreflector after undergoing two or more reflections and travels through a gas flow path 408, before emerging from window 112. The receiver unit 122 receives the light beam 410 emerging out from window 112. The invention has the benefit of ease of situating most of the equipment on a single side of the gas flow path along with the challenges associated with potential additional noise and signal loss due to the turbulent transmission path and beam steering. In addition, situating the launcher and receiver units on the same side eases the challenges associated with misalignment of launcher and receiver units.

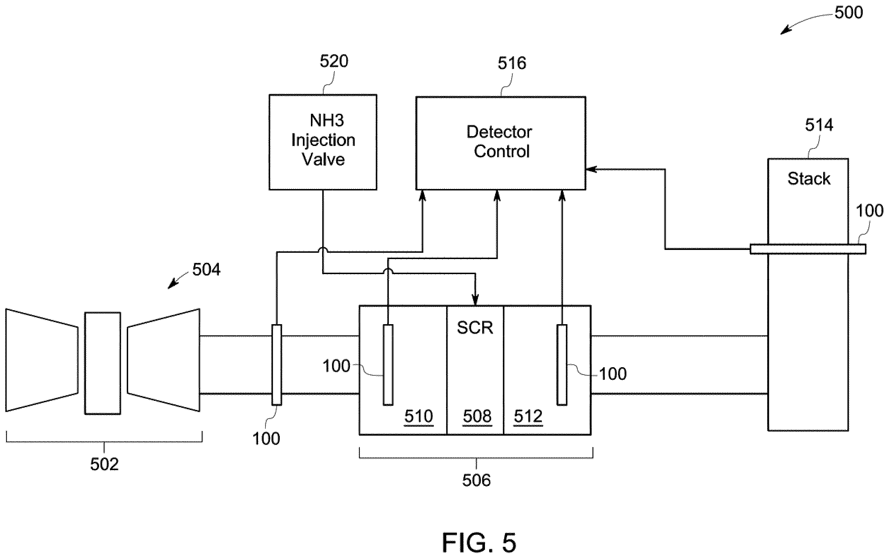

In power plants, continuous emission measurement systems (CEMS) comprising gas detectors measure and control gaseous emission species. Fig. 5 illustrates an embodiment comprising a gas turbine (GT) 502 and a gas exhaust duct 504 with a heat recovery steam generator (HRSG) 506. Some regions and states require that NOx emission from power plants be reduced to about 9-15 parts per million (ppm). It is often desirable to use catalysts in the form of selective catalytic reduction (SCR) system 508 in the HRSG to achieve these lower emission standards. In some embodiments, a plurality of gas detectors can detect concentration of gaseous species with a limit of detection as low as 1 ppm. In some embodiments, such as shown in FIG. 5, a plurality of gas detectors 100 may measure and control emission species within a gas flow path in one or more of gas turbine exhaust duct 502, inlet 510, and outlet 512 of the SCR system 508, and a gas turbine exhaust stack 514. In some other embodiments, the gas flow path being measured need not be directly in a gas turbine component. For example, some gas from one or more components may be directed to one or more sampling chambers (not shown) such that the gas flow path wherein the measurement occurs is in the one or more sampling chambers.

In a more specific embodiment, the gas flow path in the gas exhaust may comprise gaseous emission species including NO, NO2 and CO. In another specific embodiment the gas flow path at the inlet of the SCR system may comprise gaseous species including NO, NO2, NH3 and CO. In another specific embodiment in combination with or as an alternative to the aforementioned embodiment(s), the gas detector 100 may be used at the outlet 512 of SCR system 508 in HRSG 506. The gas flow path at the outlet of the SCR system may comprise at least two of the emission species NO, NO2, NH3 and CO. In one more specific embodiment, the gas flow path in the gas exhaust stack may comprise NO2, NO, NH3, CO and water molecules.

It is often desirable to operate catalysts in a narrow temperature range in which they efficiently reduce the targeted emission species such as NOx and CO. In an exemplary embodiment SCR 508 may employ NOx catalysts in a temperature range of about 600 °F to about 750 °F. In another exemplary embodiment the temperature of the gas flow path within the exhaust gas duct may be up to about 1250 °F. In yet another embodiment the temperature of the gas flow path within the exhaust stack may be about 70 °F.

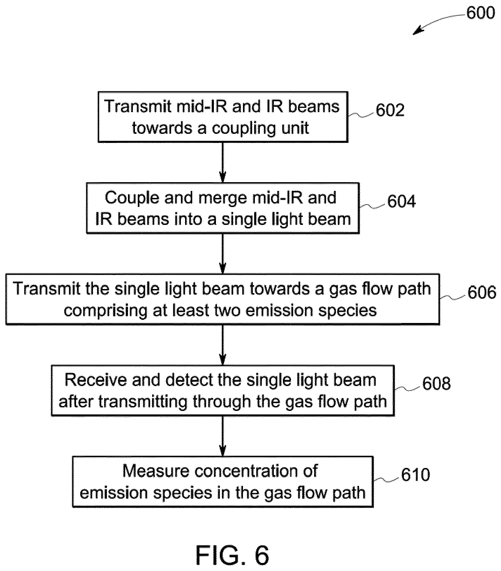

Fig. 6 illustrates a gas detection method 600 for detecting simultaneously at least two gases. At step 602, a first light beam comprising at least one mid-IR wavelength and a second light beam comprising at least one IR wavelength are transmitted towards a coupling unit. At step 604 the first and second light beams are received and coupled to produce a single coupled light beam. Further, the gas detection method includes a step 606 for transmitting the single coupled light beam towards a gas flow path. The coupled light beam interacts with gaseous species in the gas flow path while being transmitted through the gas flow path. The method 600 further includes a step 608 for receiving and detecting the light beam transmitted in step 606. In some embodiments, the gas detection method further comprises a step 610 for analyzing the detected light beam and determining concentrations of at least two gases within the gas flow path. In an exemplary embodiment, the at least two gases comprise two or more gases selected from the group of NO2, NO, NH3 and CO. In another exemplary embodiment the at least two gases comprise at least NO2 and NO. In some embodiments, the gas flow path is within a gas turbine exhaust duct, a selective catalytic reduction system, or a gas turbine exhaust stack. In some other embodiments, the gas flow path is within a sampling chamber having gaseous species extracted from one or more of a gas turbine exhaust duct, a selective catalytic reduction system, or a gas turbine exhaust stack. In some embodiments, multiple detectors may be positioned within multiple gas paths. In some embodiments, the method may further comprise a step for controlling one or more combustion emission parameters based on the measured concentration of the at least two gases. In an exemplary embodiment, one or more combustion emission parameters may include one or more of fuel-to-air ratio, flow rates, and fuel distribution.

Generally, gas turbines exhaust gas contains about 8 % to 10 % of water molecules which often results in fogging of the launching and receiving windows due to water vapor present, which may attenuate the transmitted light beam. This may lead to errors in measurement methods by increasing the absorbed signal of the transmitted beam. Another source of error in gas measurement methods includes misalignment of launcher and receiver units over a period of time, resulting in loss of detector signal over a period of time. It is therefore often desirable to detect and correct fault conditions due to measurement errors such as misalignment of launcher and receiver units or window fogging. In some embodiments, the gas detection method 600 includes a method for detecting and correcting fault conditions as described in commonly assigned US20120307241A1, resulting in reliable detection of gases. In an exemplary embodiment, the fault condition may be due to one or both of window fogging and misalignment of launcher and receiver units.

It is often desirable to minimize current noise for reducing line widths of the emitted light beams. It is often also desirable to modulate the current drive signals to the first and second sets of light sources to enable lower detection limits. In one embodiment, the control unit 124 powers and drives the first and second sets of light sources. In one specific embodiment, the control unit 124 modulates the current driving signal 160 to the first and second sets of light sources. It is often desirable to tune wavelengths of light beams from one or more light sources in first and second sets of lights sources. A DFB QCL may be tunable by 0.002 µm / K. resulting in requiring a temperature stability of 0.0001C to ensure wavelength stability. Any shift in temperature control of the laser will shift the wavelength to higher values. As a result, it is often useful to calibrate the wavelength emitted by light sources in both first and second sets of sources.

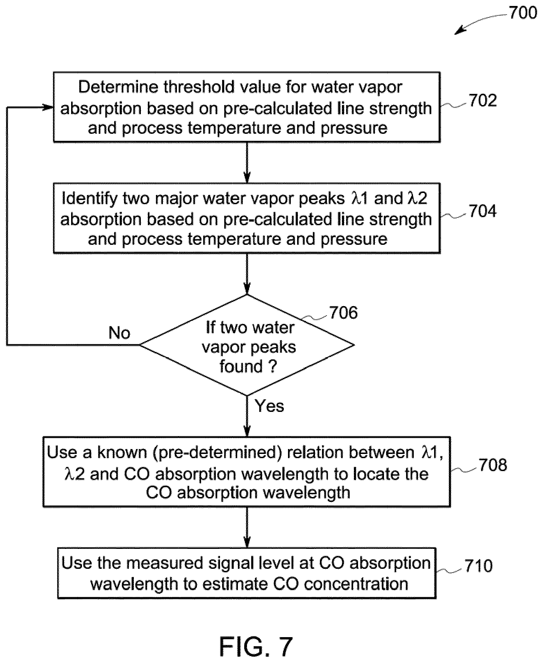

Fig. 7 illustrates an example calibration method 700 for calibrating the CO monitoring light source. At step 702, two major water vapor peaks λ₁ and λ₂ are identified such that the wavelength at which CO absorption peaks is in between λ₁ and λ₂. For example, the water vapor absorption transitions are at λ₁ = 2324nm (4304.271cm-1) and λ₂ = 2328nm (4294.647cm-1). At step 704, a pre-calculated line-strength for the water vapor lines using process temperature is used as a threshold value. For example, the pre-calculated line strength for a light source using process temperature is any value above the maximum detector current (or voltage) signal expected for the gaseous species being detected corresponding to the CO monitoring source. In step 706, in case one of the water molecule lines are not found, the light source is re-scanned with a wider scan range to locate the peaks. Once the water vapor peaks are identified, at step 708, the species peaks are located from the λ₁ peak, assuming the peak separation between the water molecules and the species peak remains same. At step 710, it is then validated with λ₂ peak of the water vapor. A similar method may be applied for calibrating each light source in the first and second sets of light sources.

## Claims

1. A gas detector (100) comprising
a launcher unit (102) comprising
a first set of light sources (202) for emitting light beams in a mid-infrared wavelength range, from 3.5 µm to 20 µm, with one of the light beams having a different wavelength than at least one other of the light beams,
a first light transmission network (204) for bundling the light beams from the first set of light sources (202) to produce a first bundled light beam (104),
a second set of light sources (212) for emitting light beams in an infrared wavelength range, from 0.8 µm to 3 µm, with one of the light beams having a different wavelength than at least one other of the light beams,
a second light transmission network (214) for bundling the light beams from the second set of light sources (212) to produce a second bundled light beam (106), and
a coupling unit (108) for receiving and coupling the first and second bundled light beams to produce a single coupled light beam (110) and for transmitting the coupled light beam towards a gas flow path (114);
a first window (112), arranged such that the coupled light beam (110) is transmitted through the first window (112) towards the gas flow path (114);
a second window (118);
a retro-reflector (404), arranged such that after interacting with the gaseous species in the gas flow path (114) the coupled light beam (402) emerges from the second window (118), is transmitted towards the retro-reflector (404), emerges from the retro-reflector (404) after undergoing two or more reflections and travels through the gas flow path, before emerging from the first window(112); and
a receiver unit (122) for receiving the coupled light beam emerging from the first window (112).

2. The gas detector (100) of claim 1, wherein the coupling unit (108) comprises a dichroic mirror (218).

3. The gas detector (100) of claim 2, wherein the dichroic mirror (218) comprises a semitransparent dichroic mirror.

4. The gas detector (100) of any of claims 1 to 3, wherein the first set of light sources (202) emit light beams at peak absorption wavelengths of at least two of NO₂ (nitrogen dioxide), NO (nitric oxide or nitrogen monoxide) and NH₃ (ammonia).

5. The gas detector (100) of any of claims 1 to 4, wherein the second set of light sources (212) is configured for emitting one of the light beams at a peak absorption wavelength of CO (carbon monoxide) and one other light beam at a wavelength insensitive to one or more emission gaseous species and water molecules in the gas flow path.

6. The gas detector (100) of any preceding claim, wherein the gas flow path (114) is within a gas turbine exhaust duct (504), a selective catalytic reduction unit (508), or a gas turbine exhaust stack (514).

7. The gas detector (100) of any preceding claim, wherein the receiver unit (122) comprises
a first dichroic beam splitter (302) for receiving the coupled light beam (120) having wavelengths in mid-infrared and infrared ranges, transmitting the light beams in the mid-infrared wavelength range (304), and reflecting the light beams in the infrared wavelength range (308), and
a second dichroic beam splitter (310) for receiving the light beams in the infrared wavelength range reflected from the first dichroic beam splitter (302), transmitting a first light beam (312), and reflecting another light beam (316) having a higher wavelength than the wavelength of the first light beam (312).

8. The gas detector (100) of claim 7, wherein the receiver unit (122) further comprises a first photo detector (306) to detect the mid-infrared light beams, a second photo detector (318) to detect the first light beam (312) transmitted from the second dichroic beam splitter (310) and a third photo detector (320) to detect the second light beam (316) reflected from the second dichroic beam splitter.

9. The gas detector (100) of claim 8, further comprising a control unit (124), wherein the control unit (124) processes the current signals (150) generated by at least the first and third photodetectors (306 and 320) to measure concentration of constituent gases in the gas flow path.

10. A method (600) for simultaneously detecting at least two gases using the gas detector (100) of any of preceding claims, the method comprising
a) transmitting (602) a first bundled light beam (104) towards a coupling unit (108), wherein the first bundled light beam (104) comprises a plurality of mid-infrared wavelengths, from 3.5 µm to 20 µm, with at least one of the plurality of mid-infrared wavelengths being different than another of the plurality of mid-infrared wavelengths;
b) transmitting (602) a second bundled light beam (106) towards the coupling unit (108), wherein the second bundled light beam (106) comprises a plurality of infrared wavelengths, from 0.8 µm to 3 µm, with at least one of the plurality of infrared wavelengths being different than another of the plurality of infrared wavelengths;
c) receiving and coupling the first and second bundled light beams (604) to produce a single coupled light beam (110);
d) transmitting the single coupled light beam (110) through a gas flow path comprising the at least two gases (606);
e) receiving and detecting the coupled light beam after the coupled beam is transmitted through the gas flow path (608) through a first window (112), emerges from a second window (118), is transmitted to a retro-reflector (404), undergoes two or more reflections in the retro-reflector (404), travels through the gas flow path and emerges from the first window (112); and
f) analyzing the detected light beam to determine concentrations of the at least two gases within the gas flow path (610).

11. The method of claim 10, wherein determining the concentrations of the at least two gases further comprises at least one of wavelength modulation spectroscopy, frequency modulation spectroscopy or direct absorption spectroscopy.

12. The method of claim 10 or 11, wherein determining the concentrations of the at least two gases further comprises calibrating the wavelength emitted by light source corresponding to each of the gaseous species being measured.

13. The method of any of claims 10 to 12, further comprising controlling one or more combustion emission parameters based at least in part on the concentration of the at least two gases.

14. The method of any of claims 10 to 13, wherein the at least the two gases comprise NO₂ (nitrogen dioxide) and NO (nitric oxide or nitrogen monoxide).

15. The method of claim 14, further comprises detecting at least one of CO (carbon monoxide) and NH₃ (ammonia).

## Patentansprüche

1. Gasdetektor (100), umfassend
eine Starteinheit (102), umfassend
einen ersten Satz von Lichtquellen (202) zum Emittieren von Lichtstrahlen in einem mittleren Infrarotwellenlängenbereich von 3,5 µm bis 20 µm, wobei einer der Lichtstrahlen eine andere Wellenlänge als mindestens ein anderer der Lichtstrahlen aufweist,
ein erstes Lichtübertragungsnetzwerk (204) zum Bündeln der Lichtstrahlen von dem ersten Satz von Lichtquellen (202), um einen ersten gebündelten Lichtstrahl (104) zu erzeugen,
einen zweiten Satz von Lichtquellen (212) zum Emittieren von Lichtstrahlen in einem Infrarotwellenlängenbereich von 0,8 µm bis 3 µm, wobei einer der Lichtstrahlen eine andere Wellenlänge als mindestens ein anderer der Lichtstrahlen aufweist,
ein zweites Lichtübertragungsnetzwerk (214) zum Bündeln der Lichtstrahlen von dem zweiten Satz von Lichtquellen (212), um einen zweiten gebündelten Lichtstrahl (106) zu erzeugen, und
eine Kopplungseinheit (108) zum Empfangen und Koppeln des ersten und des zweiten gebündelten Lichtstrahls, um einen einzelnen gekoppelten Lichtstrahl (110) zu erzeugen, und zum Übertragen des gekoppelten Lichtstrahls in Richtung eines Gasströmungspfads (114);
ein erstes Fenster (112), das so angeordnet ist, dass der gekoppelte Lichtstrahl (110) durch das erste Fenster (112) in Richtung des Gasströmungspfads (114) übertragen wird;
ein zweites Fenster (118);
einen Retroreflektor (404), der so angeordnet ist, dass nach Wechselwirkung mit der gasförmigen Spezies in dem Gasströmungspfad (114) der gekoppelte Lichtstrahl (402) aus dem zweiten Fenster (118) austritt, in Richtung des Retroreflektors (404) übertragen wird, aus dem Retroreflektor (404) austritt, nachdem er zwei oder mehr Reflexionen unterzogen wurde, und durch den Gasströmungspfad läuft, bevor er aus dem ersten Fenster (112) austritt; und
eine Empfangseinheit (122) zum Empfangen des aus dem ersten Fenster (112) austretenden gekoppelten Lichtstrahls.

2. Gasdetektor (100) nach Anspruch 1, wobei die Kopplungseinheit (108) einen dichroitischen Spiegel (218) umfasst.

3. Gasdetektor (100) nach Anspruch 2, wobei der dichroitische Spiegel (218) einen halbdurchlässigen dichroitischen Spiegel umfasst.

4. Gasdetektor (100) nach einem der Ansprüche 1 bis 3, wobei der erste Satz von Lichtquellen (202) Lichtstrahlen bei Spitzenabsorptionswellenlängen von mindestens zwei von NO₂ (Stickstoffdioxid), NO (Stickoxid oder Stickstoffmonoxid) und NH₃ (Ammoniak) emittiert.

5. Gasdetektor (100) nach einem der Ansprüche 1 bis 4, wobei der zweite Satz von Lichtquellen (212) zum Emittieren eines von den Lichtstrahlen bei einer Spitzenabsorptionswellenlänge von CO (Kohlenmonoxid) und eines anderen Lichtstrahls bei einer Wellenlänge konfiguriert ist, die gegenüber einer oder mehreren Emissionsgasspezies und Wassermolekülen in dem Gasströmungspfad unempfindlich ist.

6. Gasdetektor (100) nach einem der vorstehenden Ansprüche, wobei sich der Gasströmungspfad (114) innerhalb eines Gasturbinenabgaskanals (504), einer selektiven katalytischen Reduktionseinheit (508) oder eines Gasturbinenabgaskamins (514) befindet.

7. Gasdetektor (100) nach einem der vorstehenden Ansprüche, wobei die Empfängereinheit (122) Folgendes umfasst:
einen ersten dichroitischen Strahlteiler (302) zum Empfangen des gekoppelten Lichtstrahls (120) mit Wellenlängen im mittleren Infrarot- und Infrarotbereich, zum Übertragen der Lichtstrahlen im mittleren Infrarotwellenlängenbereich (304) und zum Reflektieren der Lichtstrahlen im Infrarotwellenlängenbereich (308) umfasst, und
einen zweiten dichroitischen Strahlteiler (310) zum Empfangen der Lichtstrahlen im Infrarotwellenlängenbereich, die vom ersten dichroitischen Strahlteiler (302) reflektiert werden, zum Übertragen eines ersten Lichtstrahls (312) und zum Reflektieren eines anderen Lichtstrahls (316) mit einer höheren Wellenlänge als die Wellenlänge des ersten Lichtstrahls (312).

8. Gasdetektor (100) nach Anspruch 7, wobei die Empfängereinheit (122) ferner einen ersten Photodetektor (306) zum Detektieren der mittleren Infrarotlichtstrahlen, einen zweiten Photodetektor (318) zum Detektieren des ersten Lichtstrahls (312), der von dem zweiten dichroitischen Strahlteiler (310) übertragen wird, und einen dritten Photodetektor (320) zum Detektieren des zweiten Lichtstrahls (316), der von dem zweiten dichroitischen Strahlteiler reflektiert wird, umfasst.

9. Gasdetektor (100) nach Anspruch 8, ferner umfassend eine Steuereinheit (124), wobei die Steuereinheit (124) die Stromsignale (150) verarbeitet, die von mindestens dem ersten und dem dritten Photodetektor (306 und 320) erzeugt werden, um die Konzentration von Bestandteilgasen in dem Gasströmungspfad zu messen.

10. Verfahren (600) zum gleichzeitigen Detektieren von mindestens zwei Gasen unter Verwendung des Gasdetektors (100) nach einem der vorstehenden Ansprüche, wobei das Verfahren Folgendes umfasst:
a) Übertragen (602) eines ersten gebündelten Lichtstrahls (104) in Richtung einer Kopplungseinheit (108), wobei der erste gebündelte Lichtstrahl (104) eine Vielzahl von mittleren Infrarotwellenlängen von 3,5 µm bis 20 µm umfasst, wobei sich mindestens eine der Vielzahl von mittleren Infrarotwellenlängen von einer anderen der Vielzahl von mittleren Infrarotwellenlängen unterscheidet;
b) Übertragen (602) eines zweiten gebündelten Lichtstrahls (106) in Richtung der Kopplungseinheit (108), wobei der zweite gebündelte Lichtstrahl (106) eine Vielzahl von Infrarotwellenlängen von 0,8 µm bis 3 µm umfasst, wobei sich mindestens eine der Vielzahl von Infrarotwellenlängen von einer anderen der Vielzahl von Infrarotwellenlängen unterscheidet;
c) Empfangen und Koppeln des ersten und des zweiten gebündelten Lichtstrahls (604), um einen einzelnen gekoppelten Lichtstrahl (110) zu erzeugen;
d) Übertragen des einzelnen gekoppelten Lichtstrahls (110) durch einen Gasströmungspfad, der die mindestens zwei Gase (606) umfasst;
e) Empfangen und Detektieren des gekoppelten Lichtstrahls, nachdem der gekoppelte Strahl durch den Gasströmungspfad (608) durch ein erstes Fenster (112) übertragen wurde, aus einem zweiten Fenster (118) austritt, zu einem Retroreflektor (404) übertragen wird, zwei oder mehr Reflexionen in dem Retroreflektor (404) unterzogen wird, durch den Gasströmungspfad läuft und aus dem ersten Fenster (112) austritt; und
f) Analysieren des detektierten Lichtstrahls, um Konzentrationen der mindestens zwei Gase innerhalb des Gasströmungspfads (610) zu bestimmen.

11. Verfahren nach Anspruch 10, wobei das Bestimmen der Konzentrationen der mindestens zwei Gase ferner mindestens eines von Wellenlängenmodulationsspektroskopie, Frequenzmodulationsspektroskopie oder Direktabsorptionsspektroskopie umfasst.

12. Verfahren nach Anspruch 10 oder 11, wobei das Bestimmen der Konzentrationen der mindestens zwei Gase ferner das Kalibrieren der von der Lichtquelle emittierten Wellenlänge entsprechend jeder der zu messenden gasförmigen Spezies umfasst.

13. Verfahren nach einem der Ansprüche 10 bis 12, ferner umfassend das Steuern eines oder mehrerer Verbrennungsemissionsparameter basierend zumindest teilweise auf der Konzentration der mindestens zwei Gase.

14. Verfahren nach einem der Ansprüche 10 bis 13, wobei die mindestens zwei Gase NO₂ (Stickstoffdioxid) und NO (Stickoxid oder Stickstoffmonoxid) umfassen.

15. Verfahren nach Anspruch 14, ferner umfassend das Detektieren von mindestens einem von CO (Kohlenmonoxid) und NH₃ (Ammoniak).

## Revendications

1. Détecteur de gaz (100) comprenant
une unité de lancement (102) comprenant
un premier ensemble de sources lumineuses (202) pour émettre des faisceaux lumineux dans une plage de longueurs d'onde de l'infrarouge moyen, de 3,5 µm à 20 µm, avec l'un des faisceaux lumineux ayant une longueur d'onde différente d'au moins un autre des faisceaux lumineux,
un premier réseau de transmission lumineuse (204) pour grouper les faisceaux lumineux du premier ensemble de sources lumineuses (202) pour produire un premier faisceau lumineux groupé (104),
un deuxième ensemble de sources lumineuses (212) pour émettre des faisceaux lumineux dans une plage de longueurs d'onde de l'infrarouge, de 0,8 µm à 3 µm, avec l'un des faisceaux lumineux ayant une longueur d'onde différente d'au moins un autre des faisceaux lumineux,
un deuxième réseau de transmission lumineuse (214) pour grouper les faisceaux lumineux du deuxième ensemble de sources lumineuses (212) pour produire un deuxième faisceau lumineux groupé (106), et
une unité de couplage (108) pour recevoir et coupler les premier et deuxième faisceaux lumineux groupés pour produire un faisceau lumineux couplé unique (110) et pour transmettre le faisceau lumineux couplé vers une voie d'écoulement de gaz (114) ;
une première fenêtre (112), agencée de telle sorte que le faisceau lumineux couplé (110) est transmis à travers la première fenêtre (112) vers la voie d'écoulement de gaz (114) ;
une deuxième fenêtre (118) ;
un rétroréflecteur (404), agencé de telle sorte qu'après avoir interagi avec les espèces gazeuses dans la voie d'écoulement de gaz (114) le faisceau lumineux couplé (402) sort de la deuxième fenêtre (118), est transmis vers le rétroréflecteur (404), sort du rétroréflecteur (404) après avoir subi deux ou plusieurs réflexions et se déplace à travers la voie d'écoulement de gaz, avant de sortir de la première fenêtre (112) ; et
une unité réceptrice (122) pour recevoir le faisceau lumineux couplé sortant de la première fenêtre (112).

2. Détecteur de gaz (100) selon la revendication 1, dans lequel l'unité de couplage (108) comprend un miroir dichroïque (218).

3. Détecteur de gaz (100) selon la revendication 2, dans lequel le miroir dichroïque (218) comprend un miroir dichroïque semi-transparent.

4. Détecteur de gaz (100) selon l'une quelconque des revendications 1 à 3, dans lequel le premier ensemble de sources lumineuses (202) émet des faisceaux lumineux à des longueurs d'onde d'absorption de crête d'au moins deux parmi le NO₂ (dioxyde d'azote), le NO (oxyde nitrique ou monoxyde d'azote) et le NH₃ (ammoniac).

5. Détecteur de gaz (100) selon l'une quelconque des revendications 1 à 4, dans lequel le deuxième ensemble de sources lumineuses (212) est configuré pour émettre l'un des faisceaux lumineux à une longueur d'onde d'absorption de crête de CO (monoxyde de carbone) et un autre faisceau lumineux à une longueur d'onde insensible à une ou plusieurs espèces gazeuses d'émission et des molécules d'eau dans la voie d'écoulement de gaz.

6. Détecteur de gaz (100) selon l'une quelconque revendication précédente, dans lequel la voie d'écoulement de gaz (114) est à l'intérieur d'un conduit d'échappement de turbine à gaz (504), d'une unité de réduction catalytique sélective (508), ou d'une cheminée d'échappement de turbine à gaz (514).

7. Détecteur de gaz (100) selon l'une quelconque revendication précédente, dans lequel l'unité réceptrice (122) comprend un premier séparateur de faisceau dichroïque (302) pour recevoir le faisceau lumineux couplé (120) ayant des longueurs d'onde dans des plages de l'infrarouge moyen et de l'infrarouge, transmettre les faisceaux lumineux dans la plage de longueurs d'onde de l'infrarouge moyen (304), et réfléchir les faisceaux lumineux dans la plage de longueurs d'onde de l'infrarouge (308), et
un deuxième séparateur de faisceau dichroïque (310) pour recevoir les faisceaux lumineux dans la plage de longueurs d'onde de l'infrarouge réfléchis par le premier séparateur de faisceau dichroïque (302), transmettre un premier faisceau lumineux (312), et réfléchir un autre faisceau lumineux (316) ayant une longueur d'onde plus élevée que la longueur d'onde du premier faisceau lumineux (312).

8. Détecteur de gaz (100) selon la revendication 7, dans lequel l'unité réceptrice (122) comprend en outre un premier photodétecteur (306) pour détecter les faisceaux lumineux de l'infrarouge moyen, un deuxième photodétecteur (318) pour détecter le premier faisceau lumineux (312) transmis par le deuxième séparateur de faisceau dichroïque (310) et un troisième photodétecteur (320) pour détecter le deuxième faisceau lumineux (316) réfléchi par le deuxième séparateur de faisceau dichroïque.

9. Détecteur de gaz (100) selon la revendication 8, comprenant en outre une unité de contrôle (124), dans lequel l'unité de contrôle (124) traite les signaux de courant (150) générés par au moins les premier et troisième photodétecteurs (306 et 320) pour mesurer une concentration de gaz constituants dans la voie d'écoulement de gaz.

10. Procédé (600) pour détecter simultanément au moins deux gaz en utilisant le détecteur de gaz (100) selon l'une quelconque des revendications précédentes, le procédé comprenant
a) la transmission (602) d'un premier faisceau lumineux groupé (104) vers une unité de couplage (108), dans lequel le premier faisceau lumineux groupé (104) comprend une pluralité de longueurs d'onde de l'infrarouge moyen, de 3,5 µm à 20 µm, avec au moins l'une de la pluralité de longueurs d'onde de l'infrarouge moyen étant différente d'une autre de la pluralité de longueurs d'onde de l'infrarouge moyen ;
b) la transmission (602) d'un deuxième faisceau lumineux groupé (106) vers l'unité de couplage (108), dans lequel le deuxième faisceau lumineux groupé (106) comprend une pluralité de longueurs d'onde de l'infrarouge, de 0,8 µm à 3 µm, avec au moins l'une de la pluralité de longueurs d'onde de l'infrarouge étant différente d'une autre de la pluralité de longueurs d'onde de l'infrarouge ;
c) la réception et le couplage des premier et deuxième faisceaux lumineux groupés (604) pour produire un faisceau lumineux couplé unique (110) ;
d) la transmission du faisceau lumineux couplé unique (110) à travers une voie d'écoulement de gaz comprenant les au moins deux gaz (606) ;
e) la réception et la détection du faisceau lumineux couplé après que le faisceau couplé est transmis à travers la voie d'écoulement de gaz (608) à travers une première fenêtre (112), sort d'une deuxième fenêtre (118), est transmis à un rétroréflecteur (404), subit deux ou plusieurs réflexions dans le rétroréflecteur (404), se déplace à travers la voie d'écoulement de gaz et sort de la première fenêtre (112) ; et
f) l'analyse du faisceau lumineux détecté pour déterminer des concentrations des au moins deux gaz à l'intérieur de la voie d'écoulement de gaz (610).

11. Procédé selon la revendication 10, dans lequel la détermination des concentrations des au moins deux gaz comprend en outre au moins l'une parmi une spectroscopie de modulation de longueur d'onde, une spectroscopie de modulation de fréquence ou une spectroscopie d'absorption directe.

12. Procédé selon la revendication 10 ou 11, dans lequel la détermination des concentrations des au moins deux gaz comprend en outre le calibrage de la longueur d'onde émise par la source lumineuse correspondant à chacune des espèces gazeuses étant mesurées.

13. Procédé selon l'une quelconque des revendications 10 à 12, comprenant en outre le contrôle d'un ou plusieurs paramètres d'émission de combustion en fonction au moins en partie de la concentration des au moins deux gaz.

14. Procédé selon l'une quelconque des revendications 10 à 13, dans lequel les au moins deux gaz comprennent du NO₂ (dioxyde d'azote) et du NO (oxyde nitrique ou monoxyde d'azote).

15. Procédé selon la revendication 14, comprend en outre la détection d'au moins l'un parmi le CO (monoxyde de carbone) et le NH₃ (ammoniac).
